(19) Europäisches Patentamt
European Patent Office
Office européen des brevets

(11) **EP 4 141 102 A1**

(12) **EUROPEAN PATENT APPLICATION**
published in accordance with Art. 153(4) EPC

(43) Date of publication:
**01.03.2023 Bulletin 2023/09**

(21) Application number: **21792725.0**

(22) Date of filing: **20.04.2021**

(51) International Patent Classification (IPC):
***C12N 1/19*** (2006.01)        ***C12N 15/113*** (2010.01)
***C12N 15/63*** (2006.01)        ***C12Q 1/6897*** (2018.01)

(52) Cooperative Patent Classification (CPC):
**C12N 15/113; C12N 15/63; C12N 15/81;**
**C12Q 1/6897**

(86) International application number:
**PCT/JP2021/016086**

(87) International publication number:
**WO 2021/215451 (28.10.2021 Gazette 2021/43)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **21.04.2020 JP 2020075196**

(71) Applicants:
• **Kumiai Chemical Industry Co., Ltd.**
**Taito-ku**
**Tokyo 110-8782 (JP)**
• **University Public Corporation Osaka**
**Osaka-shi, Osaka 545-0051 (JP)**

(72) Inventors:
• **OGAWA, Masahiro**
**Tokyo 110-8782 (JP)**
• **HARASHIMA, Sayoko**
**Sakai-shi, Osaka 599-8531 (JP)**
• **KAWANISHI, Masanobu**
**Sakai-shi, Osaka 599-8531 (JP)**
• **YAGI, Takashi**
**Sakai-shi, Osaka 599-8531 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **NUCLEIC ACID CAPABLE OF INTERACTING WITH ENDOCRINE DISRUPTOR RECEPTOR, AND USE THEREOF**

(57) This invention provides a nucleic acid that enables evaluation of various endocrine disrupting actions of a very small amount of endocrine disrupting chemicals with high sensitivity. Such nucleic acid comprises a total of 20 to 60 nucleotides comprising the nucleotide sequence shown in SEQ ID NO: 1 and shows excellent responsiveness to various endocrine disrupting chemicals.

EP 4 141 102 A1

**Description**

Technical Field

The present invention relates to a nucleic acid that interacts with a receptor for endocrine disrupting chemicals; i.e., a nucleic acid that is responsive to endocrine disrupting chemicals via a receptor for endocrine disrupting chemicals, a vector and a transformant comprising such nucleic acid, and a method for evaluating a test substance using such nucleic acid.

Background Art

Endocrine disrupting chemicals are substances that may disrupt endocrine actions of organisms and induce, for example, impairment of reproductive functions or malignant tumors. Endocrine disrupting chemicals are also referred to as "hormonally active agents." Even if the amount thereof incorporated into an organism is very small, endocrine disrupting chemicals would adversely affect normal hormone actions. Endocrine disrupting chemicals exert actions on organisms by, for example, binding to receptors to which hormones should bind in nature. Actions exerted by endocrine disrupting chemicals on organisms may be hormone-like actions or actions opposite thereto.

For example, components of agricultural chemicals, insecticides, coating materials, corrosion inhibitors, and the like include compounds that are known as endocrine disrupting chemicals exerting estrogen-like actions or androgen-like actions, and influence thereof on humans has been an issue of concern.

Meanwhile, chemicals that regulate functions of juvenile hormones (JHs) peculiar to arthropods are developed as insecticides. Juvenile hormones are characteristic hormones that regulate metamorphosis and reproduction of a variety of arthropods. When a methoprene-tolerant (MET) receptor (a Gce (germ-cell-expressed) receptor also exists in a fruit fly) accepts juvenile hormones, in the case of insects, the final form of physiological functions are exerted by activating transcription of the downstream Kruppel homolog 1 (Kr-h1). When juvenile hormones do not function normally, accordingly, arthropods having juvenile hormones cannot survive. Since mammalian animals do not have juvenile hormones, use of chemicals that regulate functions of juvenile hormones as insecticides on humans had been considered to be highly safe. There are several types of juvenile hormones. In many insects of *Coleoptera, Hymenoptera, Diptera,* and the like, JHIII is known, JH and JHII are known in *Lepidoptera,* and methyl farnesoate is known in *Crustacea.*

For example, Patent Literature 1 discloses that a response element that activates transcription of a downstream gene in response to juvenile hormones (JHs) (i.e., a juvenile hormone response element (a JH response element)) was discovered in silk worm and that a reporter assay for evaluating JH responsiveness was developed with the use of the JH response element in combination with a reporter gene. Such JH response element comprises CACGTG nucleotides referred to as an E-box. In addition, CACGCG that is located upstream of Kr-h1 and referred to as a C-box is found to function as a JH response element (Non-Patent Literature 1).

In general, daphnid produces only female offspring by parthenogenesis. Upon environmental deterioration, however, daphnid is known to produce male offspring, be fertilized, and produce eggs that are tolerant to environmental changes referred to as "resisting eggs." When daphnid is exposed to juvenile hormones or juvenile hormonally active agents or when the amount of juvenile hormones in the daphnid body is increased, *Crustacea* such as daphnid using, as a juvenile hormone, a JHIII precursor methyl farnesoate is reported to produce male offspring (Non-Patent Literature 2 and Non-Patent Literature 3). Thus, a substance that acts as a ligand to a juvenile hormone receptor or that inhibits functions of a juvenile hormone receptor can be considered as endocrine disrupting chemicals.

As a method for evaluating actions of juvenile hormones, a reproduction test that is carried out with the use of *Daphnia magna* is available. However, such reproduction test is disadvantageous in terms of the necessity of a long test period and the necessity of techniques for breeding and evaluation (Non-Patent Literature 4). While a system for evaluating actions of juvenile hormones with the use of cultured cells has been established, such system is disadvantageous in terms of the necessity of techniques for breeding and subculture. In addition, Non-Patent Literature 5 and Non-Patent Literature 6 disclose candidate nucleotide sequences of the juvenile hormone receptor gene (the MET gene) and the JH response element of *Daphnia magna,* although a practical evaluation system has not yet been developed.

Prior Art Literature

Patent Literature 1: JP Patent 5,754,681

Non-Patent Literatures

Non-Patent Literature 1: Qianyu He, et al., Heat Shock Protein 83 (Hsp83) Facilitates Methoprene-tolerant (Met) Nuclear Import to Modulate Juvenile Hormone Signaling Journal of Biological Chemistry, 289, 2014, pp. 27874-27885
Non-Patent Literature 2: Norihisa Tatarazako, et al., Juvenile hormone agonists affect the occurrence of male Daphnia, Chemosphere, 53, 2003, pp. 827-833
Non-Patent Literature 3: Kenji Toyota, et al., Methyl farnesoate synthesis is necessary for the environmental sex determination in the water flea Daphnia pulex, Journal of Insect Physiology, 80, 2015, pp. 22-30
Non-Patent Literature 4: Helen Ying Wang, et al., The screening of chemicals for juvenoid-related endocrine activity using the water flea Daphnia magna, Aquatic Toxicology 74, 2005, pp. 193-204
Non-Patent Literature 5: Tomas A. Gorr, et al., A candidate juvenoid hormone receptor cis-element in the Daphnia magna hb2 hemoglobin gene promoter, Molecular and Cellular Endocrinology 247, 2006, pp. 91-102
Non-Patent Literature 6: Nur Syafiqah Mohamad Ishak, et al., Co-option of the bZIP transcription factor Vrille as the activator of Doublesex 1 in environmental sex determination of the crustacean Daphnia magna 13, 2017, e1006953

Summary of the Invention

Objects to Be Attained by the Invention

As described above, there are endocrine disrupting chemicals for each of various hormones including juvenile hormones. Accordingly, it was necessary to prepare an evaluation system for each of various hormones to examine endocrine disrupting actions of a particular compound. In order to evaluate endocrine disrupting actions of a particular compound, as described above, a large number of steps was necessary, and a cost of evaluation was increased, disadvantageously. In addition, a very small amount of endocrine disrupting chemicals would adversely affect organisms as described above. Accordingly, development of an evaluation system that enables highly sensitive detection of endocrine disrupting actions of a very small amount of endocrine disrupting chemicals is awaited.

Under the above circumstances, it is an object of the present invention to provide a nucleic acid that can be used in a highly sensitive evaluation system that can detect various endocrine disrupting actions in a very small amount of endocrine disrupting chemicals, a vector and a transformant comprising such nucleic acid, and a method for evaluating a test substance using such nucleic acid.

Means for Attaining the Objects

The present inventors have conducted concentrated studies in order to attain the above objects. As a result, they succeeded in identifying a nucleic acid that is excellent in responsiveness to various endocrine disrupting chemicals. This has led to the completion of the present invention.

The present invention encompasses the following.

(1) A nucleic acid comprising a total of 20 to 60 nucleotides comprising the nucleotide sequence shown in SEQ ID NO: 1.
(2) The nucleic acid according to (1), wherein arbitrary 4 nucleotides (nnnn) comprised in the nucleotide sequence shown in SEQ ID NO: 1 are kmkk, provided that k indicates g or t and m indicates a or c.
(3) The nucleic acid according to (1), wherein arbitrary 4 nucleotides (nnnn) comprised in the nucleotide sequence shown in SEQ ID NO: 1 are GCGG or TATT.
(4) The nucleic acid according to any of (1) to (3), which comprises nucleotide sequences of given nucleotide lengths on the 3' terminal side and the 5' terminal side of the nucleotide sequence shown in SEQ ID NO: 1.
(5) The nucleic acid according to (1), which consists of a nucleotide sequence selected from the group consisting of SEQ ID NOs: 2, 5, 8, 34, 37, 40, 43, 46, 49, and 52.
(6) A vector comprising the nucleic acid according to any of (1) to (5).
(7) The vector according to (6), wherein the nucleic acid is designated as a unit and a plurality of units of the nucleic acids are bound to each other.
(8) The vector according to (6), which comprises a reporter gene on the 3' terminal side of a sense strand of the nucleic acid.
(9) A transformant comprising the nucleic acid according to any of (1) to (5) introduced into a host.
(10) The transformant according to (9), wherein the nucleic acid is designated as a unit and a plurality of units of the nucleic acids are bound to each other.
(11) The transformant according to (9), which comprises a reporter gene on the 3' terminal side of a sense strand of the nucleic acid.
(12) The transformant according to (9), which comprises a nucleic acid encoding a receptor for endocrine disrupting chemicals that interacts with the nucleic acid introduced thereinto.

(13) The transformant according to (12), wherein the receptor for endocrine disrupting chemicals is a juvenile hormone receptor of arthropods.

(14) The transformant according to (13), wherein the juvenile hormone receptor of arthropods is a juvenile hormone receptor of *Crustacea* or an insect.

(15) The transformant according to (14), wherein the juvenile hormone receptor of *Crustacea* is a juvenile hormone receptor of daphnid.

(16) The transformant according to (15), wherein the juvenile hormone receptor of daphnid is a protein (a) or (b):

(a) a protein consisting of the amino acid sequence shown in SEQ ID NO: 12; or
(b) a protein consisting of an amino acid sequence having 70% or higher identity to the amino acid sequence shown in SEQ ID NO: 12 and having activity of a transcription factor for a juvenile hormone receptor.

(17) The transformant according to (9), which further comprises a nucleic acid encoding a transcription-coupling factor introduced thereinto.

(18) The transformant according to (9), wherein the host is a yeast.

(19) A method for evaluating a test substance comprising:

a step of bringing a test substance into contact with a transformant comprising the nucleic acid according to any of (1) to (5) and a reporter gene on the 3' terminal side of a sense strand of the nucleic acid introduced into a host and expressing a receptor for endocrine disrupting chemicals that interacts with the nucleic acid; and
a step of assaying the expression level of the reporter gene,
wherein the interaction between the test substance and the receptor for endocrine disrupting chemicals is evaluated based on the expression level of the reporter gene.

(20) The method of evaluation according to (19), wherein, when the expression level of the reporter gene is increased after the contact with the test substance, the test substance is determined as an agonist for the receptor for endocrine disrupting chemicals.

(21) The method of evaluation according to (19), wherein the test substance is brought into contact with the transformant together with at least one substance selected from the group consisting of the endocrine disrupting chemicals interacting with the receptor for endocrine disrupting chemicals, hormones, and the agonist for the receptor for endocrine disrupting chemicals, and, when the expression level of the reporter gene is lower than the expression level measured when the substance is brought into contact by itself, the test substance is determined as an agonist for the receptor for endocrine disrupting chemicals.

(22) The method of evaluation according to (19), wherein the nucleic acid is designated as a unit and a plurality of units of the nucleic acids are bound to each other.

(23) The method of evaluation according to (19), wherein a nucleic acid encoding the receptor for endocrine disrupting chemicals is introduced into the transformant.

(24) The method of evaluation according to (19), wherein the receptor for endocrine disrupting chemicals is a juvenile hormone receptor of arthropods.

(25) The method of evaluation according to (24), wherein the juvenile hormone receptor of arthropods is a juvenile hormone receptor of *Crustacea* or an insect.

(26) The method of evaluation according to (25), wherein the juvenile hormone receptor of *Crustacea* is a juvenile hormone receptor of daphnid.

(27) The method of evaluation according to (26), wherein the juvenile hormone receptor of daphnid is a protein (a) or (b):

(a) a protein consisting of the amino acid sequence shown in SEQ ID NO: 12; or
(b) a protein consisting of an amino acid sequence having 70% or higher identity to the amino acid sequence shown in SEQ ID NO: 12 and having activity of a transcription factor for a juvenile hormone receptor.

(28) The method of evaluation according to (19), wherein the transformant further comprises a nucleic acid encoding a transcription-coupling factor introduced thereinto.

(29) The method of evaluation according to (19), wherein the host is a yeast.

(30) A kit for assaying endocrine disrupting chemicals comprising the vector according to (8) or the transformant according to (11).

Effects of the Invention

The present invention can provide a nucleic acid that is responsive to a small amount of endocrine disrupting chemicals and a vector and a transformant comprising such nucleic acid.

With the use of the nucleic acid according to the present invention, in addition, the reporter gene expression can be analyzed to evaluate the endocrine disrupting actions of the test substance.

Embodiments of the Invention

[Response element]

The nucleic acid according to the present invention (hereafter, it may be referred to as a "response element") comprises a total of 20 to 60 nucleotides comprising the nucleotide sequence shown in SEQ ID NO: 1. The term "response element" used herein does not refer to a sequence as information but the term refers to a nucleic acid comprising 4 nucleotides; i.e., adenine (A), guanine (G), thymine (T), and cytosine (C), bound to each other in a given order. Such response element interacts with a juvenile hormone receptor (an MET protein) of *Daphnia magna* bound to a juvenile hormone and positively regulates the expression of a downstream gene at the transcription level. The nucleotide sequence shown in SEQ ID NO: 1 comprises arbitrary 4 nucleotides (i.e., NNNN) between CACGCG (C-box) and CACGTG (E-box). While such arbitrary 4 nucleotides are not particularly limited, they are preferably kmkk, wherein k indicates G or T and m indicates A or C. Examples of sequences represented by kmkk include 16 types of sequences including GAGG, GCGG, TATT, TCTT, TAGG, TCGG, GATT, GCTT, and so on. Specifically, 4 nucleotides between CACGCG (C-box) and CACGTG (E-box) preferably constitute a sequence selected from among the 16 types of sequences indicated above. It is particularly preferable that 4 nucleotides between CACGCG (C-box) and CACGTG (E-box) be GCGG or TATT. A nucleotide sequence comprising GCGG as arbitrary 4 nucleotides in the nucleotide sequence shown in SEQ ID NO: 1 (i.e., CACGCGGCGGCACGTG) is included in, for example, a promoter sequence located upstream of the Vrille gene of *Daphnia magna.* Nucleotides in a region excluding SEQ ID NO: 1 of a response element can be arbitrarily selected. A full-length of a response element is composed of 20 to 60 nucleotides, preferably 30 to 60 nucleotides, more preferably 40 to 60 nucleotides, and further preferably 40 to 50 nucleotides.

The nucleotide sequence shown in SEQ ID NO: 1 (16 nucleotides) in a response element is preferably located in a region other than the both ends of a full-length sequence of 20 to 60 nucleotides. That is, a response element preferably comprises nucleotide sequences of given nucleotide lengths on the 3' terminal side and the 5' terminal side of the nucleotide sequence shown in SEQ ID NO: 1. A given nucleotide length can be composed of, for example, 1 to 43 nucleotides, preferably 5 to 30 nucleotides, more preferably 5 to 20 nucleotides, and further preferably 7 to 20 nucleotides.

A nucleotide sequence of a region excluding SEQ ID NO: 1 of a response element can be any nucleotide sequence without particular limitation. For example, a nucleotide sequence is preferably selected from a nucleotide sequence adjacent to the nucleotide sequence (i.e., CACGCGGCGGCACGTG) located in an upstream region of the Vrille gene of *Daphnia magna.*

For example, a response element comprising the nucleotide sequence shown in SEQ ID NO: 1 can be a nucleic acid consisting of a nucleotide sequence selected from the group consisting of SEQ ID NOs: 2, 5, 8, 34, and 37. In particular, a response element preferably consists of the nucleotide sequence shown in SEQ ID NO: 2.

The response element according to the present invention can be a nucleotide sequence having 75% or higher, 80% or higher, 85% or higher, 90% or higher, or 95% or higher identity to the nucleotide sequence comprising a region adjacent to the nucleotide sequence (i.e., CACGCGGCGGCACGTG) located in an upstream region of the Vrille gene of *Daphnia magna.* In particular, the nucleotide sequences shown in SEQ ID NO: 40 and SEQ ID NO: 43 comprising 3 nucleotides different from those in SEQ ID NO: 2, the nucleotide sequence shown in SEQ ID NO: 49 comprising 4 nucleotides different from those in SEQ ID NO: 2, the nucleotide sequence shown in SEQ ID NO: 46 comprising 6 nucleotides different from those in SEQ ID NO: 2, and the nucleotide sequence shown in SEQ ID NO: 52 comprising 10 nucleotides different from those in SEQ ID NO: 2 are preferable.

The response element according to the present invention positively regulates the expression of a gene located downstream of the response element (in the 3' direction of a sense strand), independently, at the transcription level. Alternatively, a plurality of response elements may be provided. Specifically, a plurality of a unit of the nucleotide sequence may be provided to be directly adjacent to each other or a plurality of units thereof may be provided via spacers. Thus, a plurality of response elements can be provided upstream of a gene whose expression is regulated at the transcription level.

By providing a plurality of response elements, more potent and positive regulation of downstream gene expression can be induced. While the number of response elements to be provided upstream of a particular gene is not particularly limited, it can be, for example, 2 to 8, preferably 2 to 5, and more preferably 3 to 4.

When a plurality of response elements are to be provided, response elements consisting of nucleotide sequences identical to each other may be provided, response elements consisting of nucleotide sequences different from each other may be provided, or response elements consisting of nucleotide sequences partially identical to each other and different from each other in other regions may be provided.

A spacer to be provided between response elements is not particularly limited, and a spacer can comprise a nucleotide sequence of 1 to a plurality of nucleotides. A nucleotide length of a spacer is not particularly limited, and a spacer can preferably comprise 1 to 100 nucleotides, 1 to 90 nucleotides, 1 to 80 nucleotides, 1 to 70 nucleotides, 1 to 60 nucleotides, 1 to 50 nucleotides, 1 to 40 nucleotides, 1 to 30 nucleotides, 1 to 20 nucleotides, or 1 to 10 nucleotides.

[Vector]

The vector according to the present invention comprises the response element described above. With the aid of the response element, the vector can incorporate a target gene whose expression is to be positively regulated at the transcription level. Specifically, the vector comprises, in a downstream region of the response element, a cloning site into which a target gene whose expression is to be positively regulated at the transcription level is to be incorporated. The vector according to the present invention is not particularly limited, provided that it can regulate downstream gene transcription with the aid of the response element in an adequate host cell. Examples of vectors include a plasmid vector, a phage vector, a cosmid vector, and a donor vector used in genome editing. In addition, a shuttle vector capable of gene exchange between host cells can be used.

Examples of plasmids include *E. coli*-derived plasmid, *Bacillus subtilis*-derived plasmid, and yeast-derived plasmid, and an example of phage is λ phage. In addition, animal virus vectors, such as retrovirus, vaccinia virus, or adenovirus vectors, and insect virus vectors, such as baculovirus vectors, can be used.

In order to insert a response element into a vector, at the outset, a purified response element is cleaved with an adequate restriction enzyme, inserted into a restriction enzyme site or a multicloning site of an adequate vector, and ligated to a vector. As a method that does not involve the use of a restriction enzyme, a homologous recombination technique, such as CRISPR/Cas9, Red/ET, or the Gateway method, is adopted.

In the vector according to the present invention, the response element is provided in an expression regulatory region that regulates downstream gene transcription. An expression regulatory region regulates transcription of a downstream gene, and it is generally located upstream of a gene (a 5' region of a sense strand). More specifically, an expression regulatory region can generally be an upstream region of several thousand nucleotides from the transcription initiation site of the downstream gene, and it can be, for example, an upstream region within 5,000 nucleotides (b), preferably 4,000 b, 3,000 b, 2,000 b, 1,000 b, 500 b, or 300 b from the transcription initiation site.

The expression regulatory region may further comprise, in addition to the response element described above, a region to which a transcription factor binds, an enhancer region, and the like. For example, an expression regulatory region comprising the response element described above can be a region of a particular length comprising such response element, such as a 50 b region, a 60 b region, a 70 b region, a 80 b region, a 90 b region, a 100 b region, a 120 b region, a 150 b region, or a 200 b region comprising such response element.

The vector according to the present invention can comprise a gene whose expression is to be regulated in a region downstream of the expression regulatory region comprising the response element constituted as described above. An example of a gene whose expression is to be regulated is a reporter gene. Examples of reporter genes include, but are not particularly limited to, the chloramphenicol acetyltransferase (CAT) gene, the lacZ gene, the luciferase gene, the β-glucuronidase (GUS) gene, the green fluorescent protein (GFP) gene, a drug-resistant gene, and an auxotrophic gene. Reporter genes are not limited to the known genes mentioned above, and the Vrille gene described above may be used as a reporter gene. Specifically, any gene can be used as a reporter gene, provided that the expression level thereof can be monitored at the transcription level.

[Transformant]

The transformant according to the present invention comprises the response element described above introduced into a host. In a transformant, for example, a juvenile hormone receptor of *Daphnia magna* bound to a juvenile hormone interacts with the response element and expression of a gene located downstream of the response element is then enhanced at the transcription level. The response element described above can be introduced into a host with the use of, for example, the vector described above. It is particularly preferable that the transformant according to the present invention comprise the response element described above, a gene whose expression is enhanced by the response element at the transcription level (e.g., a reporter gene), and a nucleic acid encoding a receptor for endocrine disrupting chemicals, such as a juvenile hormone receptor that interreacts with the response element introduced thereinto.

The term "a receptor for endocrine disrupting chemicals" used herein refers to a receptor on which a particular hormone acts as a ligand and a receptor on which an endocrine disrupting chemical having hormone-like action acts.

A receptor for endocrine disrupting chemicals encompasses both a cell membrane receptor and an intranuclear receptor. A receptor for endocrine disrupting chemicals preferably interacts with the response element described above and exerts activity of a transcription factor in the presence of a particular hormone or endocrine disrupting chemicals.

Endocrine disrupting chemicals are not particularly limited to, but refer to substances that are known to have endocrine disrupting actions or substances that are suspected of having endocrine disrupting chemicals. Examples of endocrine disrupting chemicals include substances having agonist or antagonist activity on female hormones (follicular hormones (*i.e.* estrogen) and progestational hormone (i.e. progesterone)) and substances having agonist or antagonist activity on male hormones (androgen). Endocrine disrupting chemicals are not limited to substances having disrupting action on such female or male hormones. Examples thereof include substances having disrupting actions on hormones, such as thyroid hormone, growth hormone, adrenal cortical hormone, or insulin, and neurotransmitters, such as acetylcholine, noradrenalin, adrenalin, or dopamine.

Accordingly, examples of receptors for endocrine disrupting chemicals include receptors for various hormones described above, such as an estrogen receptor, an androgen receptor, a progesterone receptor, a thyroid hormone receptor, a growth hormone receptor, an adrenal cortical hormone receptor, and an insulin receptor.

Examples of endocrine disrupting chemicals include substances having agonist or antagonist activity on arthropod-specific juvenile hormones. Accordingly, it is particularly preferable that a receptor for endocrine disrupting chemicals be a juvenile hormone receptor (MET).

Juvenile hormone receptors are not particularly limited and can be juvenile hormone receptors in arthropods, such as insects, *Crustacea, Arachnida,* and centipede. It is particularly preferable that juvenile hormone receptors in arthropods be juvenile hormone receptors in *Crustacea* or insects.

Examples of insects include *Coleoptera* including beetles and ground beetles, *Lepidoptera* including butterflies and moths, *Diptera* including flies, mosquitos, and gadflies, *Hymenoptera* including bees and ants, *Hemiptera* including cicadae and shield bugs, *Orthoptera* including locusts and crickets, and *Odonata* including dragonflies. For the transformant according to the present invention, nucleic acids encoding juvenile hormone receptors derived from such insects can be used.

More specifically, nucleic acids encoding juvenile hormone receptors of *Drosophila melanogaster, Diptera* can be used (He Q et al., J. Biol. Chem., 289 (40), pp. 27874-27885, 2014).

Examples of *Crustacea* include animals of *Crustacea,* including shrimps, crabs, krill, barnacles, and daphnids. For the transformant according to the present invention, nucleic acids encoding juvenile hormone receptors derived from such *Crustacea* can be used.

Among nucleic acids encoding juvenile hormone receptors derived from *Crustacea,* in particular, nucleic acids encoding juvenile hormone receptors derived from animals belonging to *Daphniidae* are preferably used. Examples of animals belonging to *Daphniidae* include: animals belonging to *Ceriodaphnia,* such as *Ceriodaphnia cornuta, Ceriodaphnia reticulata, Ceriodaphnia dubia, Ceriodaphnia megalops, Ceriodaphnia pulchella,* and *Ceriodaphnia* quadrangular; animals belonging to *Daphnia,* such as *Daphnia similis, Daphnia magna, Daphnia pulex, Daphnia pulicaria, Daphnia ambigua, Daphnia obtuse, Daphnia biwaensis, Daphnia longispina, Daphnia rosea, Daphnia hyaline, Daphnia galeata, Daphnia ezoensis, Daphnia cuculata, Daphnia cristata,* and *Daphnia longiremis;* animals belonging to *Scapholeberis,* such as *Scapholeberis mucronate* and *Scapholeberis kingi;* and animals belonging to *Simocephalus,* such as *Simocephalus serrulatus, Simocephalus exspinosus, Simocephalus vetulus, Simocephalus vetuloides,* and *Simocephalus japonica.*

Among them, in particular, nucleic acids encoding juvenile hormone receptors derived from animals belonging to *Daphnia* are preferably used, and nucleic acids encoding juvenile hormone receptors derived from *Daphnia magna* are more preferably used. The amino acid sequence of the juvenile hormone receptor derived from *Daphnia magna* is shown in SEQ ID NO: 12, and the nucleotide sequence of a nucleic acid encoding the juvenile hormone receptor is shown in SEQ ID NO: 11.

It should be noted that the transformant according to the present invention is not limited to a transformant comprising a nucleic acid encoding a juvenile hormone receptor consisting of the amino acid sequence shown in SEQ ID NO: 12, and the transformant may comprise a nucleic acid encoding a protein comprising an amino acid sequence having 70% or higher, preferably 80% or higher, more preferably 90% or higher, further preferably 95% or higher, and most preferably 98% or higher identity to the amino acid sequence shown in SEQ ID NO: 12 and having activity of a transcription factor for a juvenile hormone receptor.

The identity between amino acid sequences can be determined using the BLASTN or BLASTX program equipped with the Basic Local Alignment Search Tool (BLAST) algorithm (default settings). The identity is determined by subjecting a pair of amino acid sequences to pair-wise alignment analysis, calculating the number of amino acid residues completely identical between the amino acid sequences, and determining the percentage of the completely identical amino acid residues in all the amino acid residues subjected to comparison.

It should also be noted that the transformant according to the present invention is not limited to a transformant comprising a nucleic acid encoding a juvenile hormone receptor consisting of the amino acid sequence shown in SEQ ID NO: 12, and the transformant may comprise a nucleic acid encoding a protein that is encoded by a nucleic acid hybridizing under stringent conditions to a part or a full-length of a complementary strand of the nucleic acid consisting of the nucleotide sequence shown in SEQ ID NO: 11 and has activity of a transcription factor for a juvenile hormone receptor. Under "stringent conditions," a so-called specific hybrid is formed, but a non-specific hybrid is not formed. Stringent conditions can be adequately determined with reference to, for example, Molecular Cloning: A Laboratory Manual (Third Edition). Specifically, an extent of stringency can be determined by adjusting temperature and salt concentration in a solution at the time of Southern hybridization and temperature and salt concentration in a solution in the step of washing in Southern hybridization. Under stringent conditions, more specifically, sodium concentration is 25 to 500 mM and preferably 25 to 300 mM, and temperature is 42°C to 68°C and preferably 42°C to 65°C. Further specifically, sodium concentration is 5× SSC (83 mM NaCl, 83 mM sodium citrate), and temperature is 42°C.

Whether or not a protein encoded by a nucleic acid consisting of a particular nucleotide sequence other than the sequence shown in SEQ ID NO: 11 or whether or not a protein comprising amino acids different from those shown in SEQ ID NO: 12 has activity of a transcription factor for a juvenile hormone receptor can be evaluated in the manner described below. Specifically, a transformant comprising a target nucleic acid to be evaluated or a nucleic acid encoding a target protein to be evaluated, the response element described above, and a reporter gene downstream thereof introduced therein is first prepared. The transformant is then cultured in the presence or absence of juvenile hormones, and expression of the reporter gene is measured. When the expression level of the reporter gene in the presence of juvenile hormones is significantly higher than the expression level of the reporter gene in the absence of juvenile hormones, the target nucleic acid to be evaluated is determined to encode a protein having activity of a transcription factor for a juvenile hormone receptor and the target protein to be evaluated is determined to have activity of a transcription factor for a juvenile hormone receptor.

Thus, the activity of a transcription factor for a juvenile hormone receptor described above can also be referred to as activity for interacting with the response element according to the present invention. Further, the activity of a transcription factor for a juvenile hormone receptor described above can also be referred to as activity for binding to juvenile hormones and interacting with the response element according to the present invention.

In addition to the nucleic acid, the response element, and the reporter gene, the transformant according to the present invention may comprise a nucleic acid encoding a transcription-coupling factor. Examples of transcription-coupling factors include, but are not particularly limited to, Taiman (Tai), the steroid receptor coactivator (SRC), β-FTZ-F1 (fushi tarazu binding factor 1), the interacting steroid receptor coactivator (FISC), the CREB binding protein (CBP), P300, the transcriptional mediators/intermediary factor 2 (TIF2), amplified in breast cancer (AIB), the 70 kDa androgen receptor coactivator (ARA70), the activating signal co-integrator 2 (ASC2), and the 140 kDa estrogen receptor-associated protein (ERAP140). By introducing nucleic acids encoding such transcription-coupling factors, activity of a receptor for endocrine disrupting chemicals to accelerate transcription of the reporter gene can be activated.

Introduction of a nucleic acid encoding SRC or Tai is particularly preferable, and introduction of a nucleic acid encoding SRC of *Daphnia magna* is more preferable. SEQ ID NO: 14 shows the amino acid sequence of SRC of *Daphnia magna* and SEQ ID NO: 13 shows the nucleotide sequence of a nucleic acid encoding such SRC.

It should be noted that a transcription-coupling factor that can be used for the transformant according to the present invention is not limited to the protein consisting of the amino acid sequence shown in SEQ ID NO: 14, and a transcription-coupling factor may be a protein comprising an amino acid sequence having 70% or higher, preferably 80% or higher, more preferably 90% or higher, further preferably 95% or higher, and most preferably 98% or higher identity to the amino acid sequence shown in SEQ ID NO: 14 and having activity of the transcription-coupling factor.

A host of the transformant according to the present invention can be *E. coli, Bacillus subtilis,* budding yeast, an arthropod- or mammal-derived cultured cell, an undifferentiated plant cell (callus), nematode (*C. elegans*), arthropods such as daphnids or *Drosophila,* fish such as zebrafish or *Oryzias latipes,* amphibian such as *Xenopus laevis* or newt, a mammal such as a mouse or rat, or a higher plant such as *Arabidopsis thaliana* or *Oryza saliva,* with budding yeast being preferable. The above described nucleic acid or the response element, the reporter gene, the vector, and the like can be introduced into a host by any known method without particular limitation. Examples of methods for nucleic acid introduction include the lithium acetate method, the calcium phosphate method, a method involving the use of a liposome, electroporation, a method involving the use of a viral vector, and the micropipette injection method. Introduction of the above described nucleic acid or the response element, the reporter gene, and the like into the transformant according to the present invention may be transient, provided that it can be used for analysis. The above described nucleic acid or the response element, the reporter gene, the vector, and the like may be integrated into a host chromosome and introduced into the transformant. Alternatively, the above described nucleic acid or the response element, the reporter gene, the vector, and the like may be introduced into the transformant with the aid of an artificial chromosome or plasmid capable of autonomous replication and division.

In the transformant according to the present invention constituted as described above, a receptor for endocrine disrupting chemicals to which a particular hormone or endocrine disrupting chemical has bound interacts with a response element, and expression of a gene (e.g., a reporter gene) located downstream of the response element is then enhanced at the transcription level. By measuring the expression of a gene located downstream of the response element, accordingly, the interaction between the receptor for endocrine disrupting chemicals and the response element can be evaluated. On the basis of such phenomenon, the influence of a particular substance (a test substance) on the interaction between the receptor for endocrine disrupting chemicals and the response element can be evaluated. More specifically, the ability of a test substance for binding to a receptor for endocrine disrupting chemicals or the influence of a test substance on the binding between a receptor for endocrine disrupting chemicals and its agonist can be evaluated on the basis of the expression of the gene located downstream of the response element. The transformant according to the present invention involves the use of the response element described above. Thus, whether or not the test substance has endocrine disrupting actions other than the particular endocrine disrupting actions can be evaluated.

A test substance is not particularly limited, and any substance can serve as a test substance. Examples of test substances include a low-molecular-weight compound, a high-molecular-weight compound, a peptide, a single compound, a composition comprising a plurality of compounds, a culture product, an extract, a naturally-occurring substance, and a synthetic compound.

Specifically, a transformant is cultured in the presence or absence of a test substance, and the expression of a reporter gene located downstream of the response element in the presence of a test substance and that in the absence of a test substance are measured. When the expression level of the reporter gene in the presence of the test substance is significantly higher than that in the absence of the test substance, the test substance can be determined to interact with a receptor for endocrine disrupting chemicals and have agonist action to positively regulate the gene expression through the response element described above. In such a case, specifically, it is highly likely that the test substance is an endocrine disrupting chemical having agonist activity.

Alternatively, a transformant is cultured in the presence or absence of a test substance and a substance having agonist action on a receptor for endocrine disrupting chemicals, and the expression of a reporter gene located downstream of the response element in the presence of these substances and that in the absence of thee substance are measured. When the expression level of the reporter gene in the presence of the test substance is significantly lower than that in the absence of the test substance, the test substance can be determined to inhibit agonist action on a receptor for endocrine disrupting chemicals and have antagonist activity to negatively regulate the gene expression through the response element described above. In such a case, specifically, it is highly likely that the test substance is an endocrine disrupting chemical having antagonist activity. A substance exerting agonist action on a receptor for endocrine disrupting chemicals is a substance selected from among a hormone that acts on a receptor for endocrine disrupting chemicals, endocrine disrupting chemicals to such hormone, and an agonist compound of a receptor for endocrine disrupting chemicals.

With the use of the transformant according to the present invention, as described above, endocrine disrupting actions of the test substance can be evaluated via a very simple procedure. Specifically, the transformant according to the present invention can be used for measurement and evaluation of endocrine disrupting chemicals and a kit for measurement of endocrine disrupting chemicals for evaluation of endocrine disrupting actions of the test substance can be prepared.

When a transformant comprising a nucleic acid encoding a juvenile hormone receptor of *Daphnia magna* as a receptor for endocrine disrupting chemicals, the response element described above, and a reporter gene introduced thereinto is used, endocrine disrupting actions of the test substance on animals of *Daphniidae,* in particular, *Daphnia magna,* can be evaluated with high reliability.

When a yeast host is used, the transformant according to the present invention eliminates the influence, such as degradation of a test substance caused by a P450 or other drug metabolizing system of an animal cell, and enables accurate evaluation of endocrine disrupting actions of the test substance.

Examples

Hereafter, the present invention is described in greater detail with reference to the examples, although the technical scope of the present invention is not limited to the following examples.

[Preparation of yeast transformant]

In the examples, reporter plasmids, plasmids for transcription factors (receptors for endocrine disrupting chemicals), and plasmids for transcription-coupling factors were prepared as described below and introduced into budding yeast strains to prepare yeast transformants.

[Preparation of reporter plasmids]

Oligo DNA (SEQ ID NO: 3) comprising a response element consisting of the nucleotide sequence shown in SEQ ID NO: 2 (43 nucleotides) and oligo DNA (SEQ ID NO: 4) partially complementary to SEQ ID NO: 3 were prepared and annealed to each other. At the time of annealing, oligo DNAs were incubated at 95°C for 2 minutes and temperature was then gradually lowered from 90°C to 37°C over the time of 30 minutes. When two sequences are "partially complementary" to each other herein, a region excluding several nucleotides on the 5' terminal side of a nucleotide sequence identified by a sequence identification number is complementary to the same region of the other sequence.

After annealing, oligo DNA in which 1, 2, 3, 4, or 5 response elements had been consecutively annealed was purified. Subsequently, oligo DNA resulting from consecutive annealing of 1, 2, 3, 4, or 5 response elements shown in SEQ ID NO: 2 was inserted into the *Spe*I site of the pRW95-3 plasmid having the β-galactosidase gene prepared in accordance with the method described in Wolf SS et al., Biotechniques, 20 (4), pp. 568-573, 1996.

Plasmids each comprising the response element (54 nucleotides) consisting of the nucleotide sequence shown in SEQ ID NO: 5, the response element (30 nucleotides) consisting of the nucleotide sequence shown in SEQ ID NO: 8, the response element (22 nucleotides) consisting of the nucleotide sequence shown in SEQ ID NO: 34, the response element (36 nucleotides) consisting of the nucleotide sequence shown in SEQ ID NO: 37, the response element (43 nucleotides) consisting of the nucleotide sequence shown in SEQ ID NO: 40, the response element (43 nucleotides) consisting of the nucleotide sequence shown in SEQ ID NO: 43, the response element (43 nucleotides) consisting of the nucleotide sequence shown in SEQ ID NO: 46, the response element (43 nucleotides) consisting of the nucleotide sequence shown in SEQ ID NO: 49, or the response element (43 nucleotides) consisting of the nucleotide sequence shown in SEQ ID NO: 52 inserted thereinto were prepared in the same manner. Plasmids were prepared in the same manner except that oligo DNA shown in SEQ ID NO: 6, SEQ ID NO: 9, SEQ ID NO: 35, SEQ ID NO: 38, SEQ ID NO: 41, SEQ ID NO: 44, SEQ ID NO: 47, SEQ ID NO: 50, or SEQ ID NO: 53 was used instead of the oligo DNA shown in SEQ ID NO: 3, and oligo DNA partially complementary to SEQ ID NO: 6 (SEQ ID NO: 7), oligo DNA partially complementary to SEQ ID NO: 9 (SEQ ID NO: 10), oligo DNA partially complementary to SEQ ID NO: 35 (SEQ ID NO: 36), oligo DNA partially complementary to SEQ ID NO: 38 (SEQ ID NO: 39), oligo DNA partially complementary to SEQ ID NO: 41 (SEQ ID NO: 42), oligo DNA partially complementary to SEQ ID NO: 44 (SEQ ID NO: 45), oligo DNA partially complementary to SEQ ID NO: 47 (SEQ ID NO: 48), oligo DNA partially complementary to SEQ ID NO: 50 (SEQ ID NO: 51), or oligo DNA partially complementary to SEQ ID NO: 53 (SEQ ID NO: 54) was used instead of the oligo DNA shown in SEQ ID NO: 4. Thus, a plasmid comprising 3 consecutive repeats of the response element shown in SEQ ID NO: 5 inserted thereinto, a plasmid comprising 1, 2, 3, or 4 consecutive repeats of the response element shown in SEQ ID NO: 8 inserted thereinto, a plasmid comprising 1, 2, 3, 4, or 5 consecutive repeats of the response element shown in SEQ ID NO: 34 inserted thereinto, a plasmid comprising 1, 2, 3, or 4 consecutive repeats of the response element shown in SEQ ID NO: 37 inserted thereinto, a plasmid comprising 1, 2, 3, or 4 consecutive repeats of the response element shown in SEQ ID NO: 40 inserted thereinto, a plasmid comprising 1, 2, 3, or 4 consecutive repeats of the response element shown in SEQ ID NO: 43 inserted thereinto, a plasmid comprising 1, 2, or 3 consecutive repeats of the response element shown in SEQ ID NO: 46 inserted thereinto, a plasmid comprising 1, 2, 3, or 4 consecutive repeats of the response element shown in SEQ ID NO: 49 inserted thereinto, and a plasmid comprising 1, 2, or 3 consecutive repeats of the response element shown in SEQ ID NO: 52 inserted thereinto were prepared. The nucleotide sequences shown in SEQ ID NO: 2, SEQ ID NO: 5, SEQ ID NO: 8, SEQ ID NO: 34, and SEQ ID NO: 37 are located upstream of the Vrille gene of *Daphnia magna,* the nucleotide sequence shown in SEQ ID NO: 40 is derived from the nucleotide sequence shown in SEQ ID NO: 2 by substitution of 3 nucleotides outside the 5' terminus of the C-box (CACGCG), the nucleotide sequence shown in SEQ ID NO: 43 is derived from the nucleotide sequence shown in SEQ ID NO: 2 by substitution of 3 nucleotides outside the 3' terminus of the E-box (CACGTG), the nucleotide sequence shown in SEQ ID NO: 46 is derived from the nucleotide sequence shown in SEQ ID NO: 2 by substitution of 3 nucleotides outside the 5' terminus of the C-box (CACGCG) and 3 nucleotides outside the 3' terminus of the E-box (CACGTG), the nucleotide sequence shown in SEQ ID NO: 49 is derived from the nucleotide sequence shown in SEQ ID NO: 2 by substitution of 4 nucleotides in a region (a linker region) between the C-box (CACGCG) and the E-box (CACGTG), and the nucleotide sequence shown in SEQ ID NO: 52 is derived from the nucleotide sequence shown in SEQ ID NO: 2 by substitution of 3 nucleotides outside the 5' terminus of the C-box (CACGCG), 3 nucleotides outside the 3' terminus of the E-box (CACGTG), and 4 nucleotides in a region (a linker region) between the C-box (CACGCG) and the E-box (CACGTG).

For comparison with the reporter plasmid comprising the response element, a plasmid comprising the nucleic acid consisting of 16 nucleotides (i.e., CACGCGGCGGCACGTG) located in an upstream region of the Vrille gene of *Daphnia magna* inserted thereinto was prepared. Such 16 nucleotides include the E-box (CACGTG) and the C-box (CACGCG) serving as juvenile hormone response elements in various insects. In accordance with the method for preparing plasmids described above, a plasmid comprising 1, 2, 3, or 4 consecutive repeats of the nucleic acid consisting of 16 nucleotides inserted thereinto was prepared.

For further comparison, a plasmid comprising a region including the juvenile hormone response element of the Kr-h1 gene of *Drosophila melanogaster* (DmJHRR) inserted thereinto was prepared. DmJHRR is described in He Q et al., J. Biol. Chem., 289 (40), pp. 27874-27885, 2014. A plasmid comprising 3 consecutive repeats of the DmJHRR sequence inserted thereinto was prepared in accordance with the method described above, except that oligo DNA comprising the DmJHRR sequence (SEQ ID NO: 15) was used instead of the oligo DNA shown in SEQ ID NO: 3 and oligo DNA partially complementary to SEQ ID NO: 15 (SEQ ID NO: 16) was used instead of the oligo DNA shown in SEQ ID NO: 4.

In order to compare reporter activity influenced by the presence or absence of the C-box (CACGCG) and the E-box (CACGTG) in the nucleotide sequence shown in SEQ ID NO: 2, a nucleotide sequence having a C-box different from the C-box in SEQ ID NO: 2 (SEQ ID NO: 25), a nucleotide sequence having an E-box different from the E-box in SEQ ID NO: 2 (SEQ ID NO: 28), and a nucleotide sequence having a C-box and an E-box different from the C-box and the E-box in SEQ ID NO: 2 (SEQ ID NO: 31) were prepared. Specifically, oligo DNA comprising the nucleotide sequence shown in SEQ ID NO: 25 (SEQ ID NO: 26) and DNA partially complementary to SEQ ID NO: 26 (SEQ ID NO: 27), oligo DNA comprising the nucleotide sequence shown in SEQ ID NO: 28 (SEQ ID NO: 29) and DNA partially complementary to SEQ ID NO: 29 (SEQ ID NO: 30), and oligo DNA comprising the nucleotide sequence shown in SEQ ID NO: 31 (SEQ ID NO: 32) and DNA partially complementary to SEQ ID NO: 32 (SEQ ID NO: 33) were prepared, and a plasmid comprising 4 consecutive repeats of the nucleotide sequence shown in SEQ ID NO: 25 inserted thereinto, a plasmid comprising 4 consecutive repeats of the nucleotide sequence shown in SEQ ID NO: 28 inserted thereinto, and a plasmid comprising 4 consecutive repeats of the nucleotide sequence shown in SEQ ID NO: 31 inserted thereinto were prepared.

[Preparation of a plasmid expressing a transcription factor]

In order to prepare a plasmid expressing a juvenile hormone receptor of *Daphnia magna* (hereafter, referred to as a "DampaMET plasmid"), a CEN6/ARS4 fragment was first prepared. A DNA fragment comprising the autonomously replicating sequence (ARS) and the centromere sequence (CEN) of yeast was amplified by PCR with the use of, as a template, the pYTβ reporter plasmid and the primers for CEN/ARS amplification: pUdp6 I-SceI CEN6 FW (SEQ ID NO: 17) and pUdp6 I-SceI ARS4 RE (SEQ ID NO: 18). PCR was carried out by repeating a cycle of 94°C for 20 seconds, 58°C for 20 seconds, and 72°C for 1.5 minutes 35 times.

A PCR-amplified fragment and the pUdp6 plasmid linearized by cleavage with the *Aat*II restriction enzyme were introduced into wild-type yeast strains (*Saccharomyces cerevisiae*) W303a (MATa, ade2, his3, leu2, trp1, ura3) by the lithium acetate method. The pUdp6 plasmid has a bidirectional promoter regions gal1 and gal10, a CYC terminator downstream of gal10, an ADH terminator downstream of gal1, and the uracil selection marker URA3 gene. A plasmid comprising the CEN6/ARS4 fragment inserted into pUdp6 via homologous recombination was extracted from yeast and introduced into the *E. coli* DH5α strain to prepare a low-copy episomal vector pUdp13. A recognition sequence of the 18-bp I-SceI restriction enzyme has been introduced into a site outside the CEN6/ARS4 sequence. After the target gene is cloned into the multicloning site, accordingly, the CEN6/ARS4 fragment is cleaved with I-SceI, and the resulting fragment can be readily converted into a genome insertion-type plasmid.

Subsequently, an open reading frame (ORF) of cDNA of the DampaMET gene was amplified by PCR using, as a template, cDNA of adult *Daphnia magna* carrying a resting egg and the primers for DampaMET: MET FW2 (SEQ ID NO: 19) and MET REV-3 (SEQ ID NO: 20). PCR was carried out by repeating a cycle of 94°C for 20 seconds, 58°C for 20 seconds, and 72°C for 2.5 minutes 35 times.
cDNA of the amplified DampaMET gene and the pUdp13 plasmid cleaved with the *Bam*HI and *Hind*III restriction enzymes were introduced into the wild-type yeast strain W303a by the lithium acetate method. A plasmid prepared by this method, which comprises cDNA of the DampaMET gene inserted into a site downstream of the GAL 10 promoter of the pUdp13 plasmid, was designated as pUdp13-DapmaMet. pUdp13-DapmaMet constructed in a yeast cell was recovered, introduced into the *E. coli* DH5α strain, and amplified.

Subsequently, pUdp13-DapmaMet was cleaved with the I-SceI restriction enzyme to obtain the CEN6/ARS4 sequence and converted into a genome insertion-type plasmid. Thus, the DampaMET plasmid comprising the DampaMET gene and a promoter upstream thereof was obtained.

A plasmid expressing a juvenile hormone receptor of *Drosophila melanogaster* (hereafter, referred to as a "DmMET plasmid") was prepared in the same manner. At the outset, ORF of cDNA of the DmMET gene was amplified by PCR using, as a template, cDNA of *Drosophila melanogaster* larvae (Clontech) and the primers for DmMET: DmMET Fwd (SEQ ID NO: 21) and DmMET Rev (SEQ ID NO: 22). PCR was carried out by repeating a cycle of 98°C for 10 seconds, 55°C for 30 seconds, and 68°C for 1.5 minutes 25 times.
cDNA of the amplified DmMET gene was cleaved with the *Sma*I and *Eco*RI restriction enzymes. cDNA of the cleaved DmMET gene was inserted into a region downstream of the GAL10 promoter of the pUdp6 plasmid cleaved with the *Sma*I and *Eco*RI restriction enzymes. Thus, the DmMET plasmid comprising the DmMET gene and a promoter upstream thereof was obtained.

[Preparation of a plasmid expressing a transcription-coupling factor]

In the example, in particular, a plasmid expressing a transcription factor comprising the *Drosophila melanogaster* Tai (DmTai) gene (hereafter, referred to as a "DmTai plasmid") and a plasmid expressing a *Daphnia magna* transcription-coupling factor (SRC) (hereafter, referred to as a "DampaSRC plasmid") were prepared and used. The DmTai plasmid was prepared in accordance with Ito-Harashima S et al., FEBS Open Bio., 7 (7): pp. 995-1008, 2017.

The DampaSRC plasmid was prepared in the manner described below. At the outset, ORF of cDNA of the *Daphnia magna* SRC (DampaSRC) gene was amplified by PCR using, as a template, cDNA of adult *Daphnia magna* carrying a resting egg and the primers for SRC: SRC-F1F-pESC (SEQ ID NO: 23) and SRC-F4short-pESC (SEQ ID NO: 24). PCR was carried out by repeating a cycle of 94°C for 20 seconds, 58°C for 10 seconds, and 72°C for 7 minutes 35 times.

Subsequently, the pESC-Leu plasmid (Agilent technology) was cleaved with the *Spe*I and *Pac*I restriction enzymes, and cDNA of the amplified DampaSRC gene was introduced into the wild-type yeast strain W303a by the lithium acetate method. Thus, the DampaSRC plasmid comprising cDNA of the DampaSRC gene inserted downstream of the gal1 promoter region of the pESC-Leu plasmid was obtained. The DampaSRC plasmid constructed in a yeast cell was recovered, introduced into the *E. coli* DH5α strain, and amplified.

[Preparation of yeast transformant]

At the outset, the cells of the budding yeast strain W303a were cultured in a YPD medium (1% yeast extract, 2% peptone, 2% D (+)-glucose) at 30°C until the turbidity (O.D. 595) reached 0.7 to 0.8. The cultured cells were washed 2 times with sterile water and resuspended in a 0.1 mol/l lithium acetate solution in an amount of 1/10 of the culture solution using a pipette. The cell suspension was fractionated in an amount of 100 μl each to 1.5-ml microtubes, and the supernatant was removed via centrifugation. A TE buffer (75 μl; 10 mM Tris, 1 mM EDTA, pH 8.0) containing 1 μg of the DampaMET plasmid linearized via treatment with the EcoRV restriction enzyme and 50 μg of carrier DNA (tradename: SALMON TESTESDNA for hybridization, SIGMA) was added, and 240 μl of a 50% polyethylene glycol 3350 solution and 36 μl of a 0.1 mol/l lithium acetate solution were further added, followed by thorough mixing.

The mixture was incubated at 30°C for 30 minutes, heat-treated at 42°C for 22 minutes, and centrifuged at 9,000 rpm for 1 minute to harvest yeast cells from the mixture. The harvested yeast cells were suspended in 300 μl of sterile water, 100 ml of the suspension was applied to a selection medium prepared by adding 42 mg of tryptophan, 62 mg of leucine, and 2% agarose to the medium shown in Table 1 and Table 2, and culture was then performed. Uracil-nonrequiring yeast strains were selected as yeast transformants comprising the gal1 promoter and the cDNA region of the MET gene integrated into the chromosome.

Subsequently, the DampaSRC plasmid was introduced into the yeast transformant comprising the DampaMET plasmid introduced thereinto. As a selection medium for the yeast transformant, a culture medium prepared by adding 100 mg of tryptophan to the culture medium shown in Table 1 and Table 2 was used.

[Table 1]

| Composition of pre-culture medium | |
|---|---|
| Components | Amount |
| Yeast nitrogen base w/o amino acids and ammonium sulfate | 1.7 g |
| $(NH_4)_2SO_4$ | 5 g |
| Drop-out powder (Table 2) | 1.3 g |
| 5M NaOH | 500 μl |
| D(+)-glucose | 20 g |
| Water | 1 l |
| Solid medium was prepared with the addition of 2% agar to the above composition, autoclaving, and transfer to a petri dish | |

[Table 2]

| Composition of drop-out powder | |
|---|---|
| Components | Amount |
| Adenine | 2.5 g |

(continued)

| Composition of drop-out powder | |
|---|---|
| Components | Amount |
| L-Aspartic acid | 6.0 g |
| L-Histidine | 1.2 g |
| L-Arginine-HCl | 1.2 g |
| L-Methionine | 1.2 g |
| L-Lysine-HCl | 1.8 g |
| L-Glutamic acid | 6.0 g |
| L-Valine | 9.0 g |
| L-Serine | 22.5 g |
| L-Threonine | 12.0 g |

Subsequently, a reporter plasmid prepared by inserting the nucleotide sequence shown in SEQ ID NO: 2 into a yeast comprising the DampaMET plasmid and the DampaSRC plasmid introduced thereinto by the lithium acetate method. As a selection medium, the pre-culture medium shown in Table 1 and Table 2 was used. Thus, Yeast 1 of Invention was obtained. In the same manner, Yeasts 2 to 38 of Invention each comprising the response element according to the present invention and Control yeasts 1 to 9 shown in Table 3 were prepared.

[Table 3]

| | Response element type | Response element unit | Transcription factor | Coupling factor |
|---|---|---|---|---|
| Yeast 1 of Invention | SEQ ID NO: 2 | 1 | DampaMET | DampaSRC |
| Yeast 2 of Invention | SEQ ID NO: 2 | 2 | DampaMET | DampaSRC |
| Yeast 3 of Invention | SEQ ID NO: 2 | 3 | DampaMET | DampaSRC |
| Yeast 4 of Invention | SEQ ID NO: 2 | 4 | DampaMET | DampaSRC |
| Yeast 5 of Invention | SEQ ID NO: 2 | 5 | DampaMET | DampaSRC |
| Yeast 6 of Invention | SEQ ID NO: 5 | 3 | DampaMET | DampaSRC |
| Yeast 7 of Invention | SEQ ID NO: 8 | 3 | DampaMET | DampaSRC |
| Yeast 8 of Invention | SEQ ID NO: 2 | 4 | DmMET | DmTai |
| Yeast 9 of Invention | SEQ ID NO: 34 | 1 | DampaMET | DampaSRC |
| Yeast 10 of Invention | SEQ ID NO: 34 | 2 | DampaMET | DampaSRC |
| Yeast 11 of Invention | SEQ ID NO: 34 | 3 | DampaMET | DampaSRC |
| Yeast 12 of Invention | SEQ ID NO: 34 | 4 | DampaMET | DampaSRC |
| Yeast 13 of Invention | SEQ ID NO: 34 | 5 | DampaMET | DampaSRC |
| Yeast 14 of Invention | SEQ ID NO: 8 | 1 | DampaMET | DampaSRC |
| Yeast 15 of Invention | SEQ ID NO: 8 | 2 | DampaMET | DampaSRC |
| Yeast 16 of Invention | SEQ ID NO: 8 | 4 | DampaMET | DampaSRC |
| Yeast 17 of Invention | SEQ ID NO: 37 | 1 | DampaMET | DampaSRC |
| Yeast 18 of Invention | SEQ ID NO: 37 | 2 | DampaMET | DampaSRC |
| Yeast 19 of Invention | SEQ ID NO: 37 | 3 | DampaMET | DampaSRC |
| Yeast 20 of Invention | SEQ ID NO: 37 | 4 | DampaMET | DampaSRC |
| Yeast 21 of Invention | SEQ ID NO: 40 | 1 | DampaMET | DampaSRC |

(continued)

| | Response element type | Response element unit | Transcription factor | Coupling factor |
|---|---|---|---|---|
| Yeast 22 of Invention | SEQ ID NO: 40 | 2 | DampaMET | DampaSRC |
| Yeast 23 of Invention | SEQ ID NO: 40 | 3 | DampaMET | DampaSRC |
| Yeast 24 of Invention | SEQ ID NO: 40 | 4 | DampaMET | DampaSRC |
| Yeast 25 of Invention | SEQ ID NO: 43 | 1 | DampaMET | DampaSRC |
| Yeast 26 of Invention | SEQ ID NO: 43 | 2 | DampaMET | DampaSRC |
| Yeast 27 of Invention | SEQ ID NO: 43 | 3 | DampaMET | DampaSRC |
| Yeast 28 of Invention | SEQ ID NO: 43 | 4 | DampaMET | DampaSRC |
| Yeast 29 of Invention | SEQ ID NO: 46 | 1 | DampaMET | DampaSRC |
| Yeast 30 of Invention | SEQ ID NO: 46 | 2 | DampaMET | DampaSRC |
| Yeast 31 of Invention | SEQ ID NO: 46 | 3 | DampaMET | DampaSRC |
| Yeast 32 of Invention | SEQ ID NO: 49 | 1 | DampaMET | DampaSRC |
| Yeast 33 of Invention | SEQ ID NO: 49 | 2 | DampaMET | DampaSRC |
| Yeast 34 of Invention | SEQ ID NO: 49 | 3 | DampaMET | DampaSRC |
| Yeast 35 of Invention | SEQ ID NO: 49 | 4 | DampaMET | DampaSRC |
| Yeast 36 of Invention | SEQ ID NO: 52 | 1 | DampaMET | DampaSRC |
| Yeast 37 of Invention | SEQ ID NO: 52 | 2 | DampaMET | DampaSRC |
| Yeast 38 of Invention | SEQ ID NO: 52 | 3 | DampaMET | DampaSRC |
| Control yeast 1 | cacgcggcggcacgtg | 1 | DampaMET | DampaSRC |
| Control yeast 2 | cacgcggcggcacgtg | 2 | DampaMET | DampaSRC |
| Control yeast 3 | cacgcggcggcacgtg | 3 | DampaMET | DampaSRC |
| Control yeast 4 | cacgcggcggcacgtg | 4 | DampaMET | DampaSRC |
| Control yeast 5 | DmJHRR | 3 | DampaMET | DmTai |
| Control yeast 6 | DmJHRR | 3 | DmMET | DmTai |
| Control yeast 7 | SEQ ID NO: 25 | 4 | DampaMET | DampaSRC |
| Control yeast 8 | SEQ ID NO: 28 | 4 | DampaMET | DampaSRC |
| Control yeast 9 | SEQ ID NO: 31 | 4 | DampaMET | DampaSRC |

The test examples described below were performed using the yeast transformants.

[Test Example 1] Assay of reporter activity of yeast transformant to juvenile hormones

With the use of the Yeast 1 of Invention, reporter activity to methyl farnesoate as a juvenile hormone substance was measured. At the outset, the Yeast 1 of Invention was subjected to pre-culture at 30°C for 18 hours using the pre-culture medium shown in Table 1 until the turbidity (O.D. 595) of the pre-culture medium reached approximately 0.1. The solution of the pre-cultured cells (10 μl) and 1 μl of the solution of methyl farnesoate diluted to various concentrations with dimethyl sulfoxide (DMSO) were mixed with 90 μl of the main culture solution shown in Table 4 in a 96-well plate, and the mixture was subjected to static culture at 30°C for 18 hours to prepare a reaction solution. The resultant was designated as a test group.

[Table 4]

| Composition of main culture medium | |
|---|---|
| Components | Amount |
| Yeast nitrogen base w/o amino acids and ammonium sulfate | 1.7 g |
| $(NH_4)_2SO_4$ | 5.0 g |
| Drop-out powder (Table 2) | 1.3 g |
| 5M NaOH | 500 μl |
| D(+)-galactose | 10 g |
| Water | 1 l |

A reaction solution prepared with the addition of 1 μl of DMSO instead of a diluted methyl farnesoate solution was designated as a control group. Fractions of 10 μl each were collected from the reaction solutions and dispensed into each well of another 96-well plate. Thereafter, 100 μl of an assay reagent comprising a lytic solution (Z buffer: 60 mM $Na_2HPO_4$, 40 mM $NaH_2PO_4$, 1 mM $MgCl_2$, 10 mM KCl, 2 mM dithiothreitol, 0.20% N-lauroylsarcosine sodium salt) mixed with 1 mg/ml ONPG (orthonitrophenylgalactopyranoside) was dispensed into each well, and the reaction was allowed to proceed at 37°C for 30 minutes. Thereafter, the absorbance (O.D. 405) and the turbidity (O.D. 595) of the reaction solutions were assayed at the wavelength of 405 nm using a microplate reader (tradename: iMark microplate reader, Bio-Rad Laboratories). The increase of induction was calculated in accordance with the following formula.

$$\text{Increase in induction} \quad = \quad \frac{O.D.405 \text{ of Test group}}{O.D.595 \text{ of Test group}} \quad - \quad \frac{O.D.405 \text{ of Control group}}{O.D.595 \text{ of Control group}}$$

The increase of induction is described in Ito-Harashima S et al., FEBS Open Bio., 7 (7): pp. 995-1008, 2017. When the increase of induction is positive, the transformant is determined to have reporter activity to the substance tested. The test was performed once or repeated 3 times, 5 times, or 15 times, and the average increase of induction was calculated. The control yeast 1 was treated in the same manner and reporter activity was compared. The results are shown in Table 5.

[Table 5]

| Tested yeast transformant | Amount of juvenile hormone (methyl farnesoate) treated | |
|---|---|---|
| | 100 pM | 1 nM |
| Yeast 1 of Invention | 0.10 | 0.08 |
| Control yeast 1 | 0.01 | 0.00 |

As shown in Table 5, reporter gene expression is induced in the presence of methyl farnesoate, which is a juvenile hormone, in the Yeast 1 of Invention comprising the response element. In the control yeast 1, in contrast, substantially no reporter activity was detected.

[Test Example 2] Assay of reporter activity of yeast transformant to juvenile hormones

In the same manner as in Test Example 1, the Yeasts 2, 3, 4, 5, 6, 7, 9, 10, 11, 12, 13, 14, 15, 16, 17, 18, 19, and 20 of Invention were subj ected to assays of reporter activity to methyl farnesoate. The test was performed in accordance with Test Example 1, and reporter activity of the yeasts indicated above was compared with that of the control yeasts 2, 3, and 4. The results are shown in Table 6 and Table 7.

[Table 6]

| Tested yeast transformant | Amount of juvenile hormone (methyl farnesoate) treated | | | | |
|---|---|---|---|---|---|
| | 10 nM | 100 nM | 1 $\mu$M | 10 $\mu$M | 100 $\mu$M |
| Yeast 2 of Invention | 0.54 | 1.26 | 1.45 | 1.64 | 1.57 |
| Yeast 3 of Invention | 2.05 | 4.35 | 4.45 | 4.07 | 4.29 |
| Yeast 4 of Invention | 5.13 | 7.55 | 7.12 | 7.44 | 7.47 |
| Yeast 5 of Invention | 1.33 | 3.29 | 3.72 | 3.76 | 4.05 |
| Yeast 6 of Invention | 0.33 | 1.19 | 1.18 | 1.33 | 1.33 |
| Yeast 7 of Invention | 0.06 | 0.26 | 0.32 | 0.36 | 0.34 |
| Control yeast 2 | -0.29 | -0.27 | -0.34 | -0.21 | -0.14 |
| Control yeast 3 | -0.12 | -0.21 | -0.27 | -0.17 | -0.17 |
| Control yeast 4 | -0.84 | -1.49 | -1.82 | -1.05 | -2.06 |

[Table 7]

| Tested yeast transformant | Amount of juvenile hormone (methyl farnesoate) treated | |
|---|---|---|
| | 100 nM | 10 $\mu$M |
| Yeast 9 of Invention | 0.19 | 0.16 |
| Yeast 10 of Invention | 0.08 | 0.20 |
| Yeast 11 of Invention | 0.22 | 0.24 |
| Yeast 12 of Invention | 1.43 | 2.55 |
| Yeast 13 of Invention | - | 0.09 |
| Yeast 14 of Invention | - | 0.09 |
| Yeast 15 of Invention | 0.09 | 0.16 |
| Yeast 16 of Invention | 0.65 | 1.32 |
| Yeast 17 of Invention | 0.06 | 0.09 |
| Yeast 18 of Invention | 0.10 | - |
| Yeast 19 of Invention | 1.53 | 227 |
| Yeast 20 of Invention | 1.55 | 1.85 |

As shown in Table 6 and Table 7, it is apparent that potent expression of the reporter gene is induced in the presence of a juvenile hormone (methyl farnesoate) in the Yeasts 2, 3, 4, 5, 6, 7, 9, 10, 11, 12, 13, 14, 15, 16, 17, 18, 19, and 20 of Invention. In contrast, the increase of induction was negative in the control yeasts 2, 3, and 4 in any amount of treatment, and such control yeasts did not show any reporter activity. The results demonstrate that use of a nucleotide sequence comprising a total of 20 to 60 nucleotides including the nucleotide sequence shown in SEQ ID NO: 1 as a response element would enable evaluation of reporter activity to methyl farnesoate, which is a juvenile hormone, with high sensitivity.

[Test Example 3] Influence of core sequence on reporter activity of yeast transformant

With the use of the control yeast 7 comprising a different nucleotide sequence of the C-box (CACGCG) in the response element, the control yeast 8 comprising a different nucleotide sequence of the E-box (CACGTG), and the control yeast 9 comprising different nucleotide sequences of the C-box and the E-box from the relevant nucleotide sequences of the Yeast 4 of Invention, the influence of a core sequence on reporter activity to methyl farnesoate was evaluated. The test was performed in accordance with Test Example 1. The results are shown in Table 8.

[Table 8]

| Tested yeast transformant | Amount of juvenile hormone (methyl farnesoate) treated | |
|---|---|---|
| | 100 nM | 10 $\mu$M |
| Yeast 4 of Invention | 0.42 | 0.51 |
| Control yeast 7 | -0.42 | -0.12 |
| Control yeast 8 | 0.00 | -0.02 |
| Control yeast 9 | -0.42 | -0.12 |

As shown in Table 8, high reporter activity to methyl farnesoate was induced in the Yeast 4 of Invention. In the control yeasts 7, 8, and 9 each comprising different nucleotide sequences in the C-box and/or the E-box, in contrast, reporter activity was lowered to a significant extent, compared to the Yeast 4 of Invention.

[Test Example 4] Assay of reporter activity of yeast transformant to sex hormones and chemical substances suspected of having endocrine disrupting actions

With the use of the Yeast 4 and the Yeast 8 of the present Invention, reporter activity to sex hormones; i.e., 17$\beta$-estradiol and testosterone, insect juvenile hormones; i.e., juvenile hormone III, and chemical substances suspected of having endocrine disrupting actions, such as 4-nonyl phenol, bisphenol A, 1,1,1-trichloro-2,2-bis(4-chlorophenyl)ethane (DDT), 2,2-bis(4-chlorophenyl)-1,1-dichloroethylene (DDE), and dieldrin, was assayed. The test was performed in accordance with Test Example 1. All the test substances were treated at 10 $\mu$M. For comparison, reporter activity to compounds was assayed using the control yeast 5 and the control yeast 6 comprising a known juvenile hormone receptor response element (i.e., the JH response element of *Drosophila melanogaster*) integrated therein. The results are shown in Table 9.

[Table 9]

| Tested yeast transformant | Increase in induction | | | | | | | |
|---|---|---|---|---|---|---|---|---|
| | 17$\beta$-estradiol | Testosterone | Juvenile hormone III | 4-nonyl phenol | Bisphenol A | DDT | DDE | Dieldrin |
| Yeast 4 of Invention | 1.97 | 1.78 | 6.20 | 2.16 | 1.14 | 0.65 | 2.15 | 3.85 |
| Yeast 8 of Invention | 1.01 | 0.98 | 1.45 | 1.81 | 0.52 | 0.37 | 1.23 | 0.89 |
| Control yeast 5 | -0.14 | -0.02 | 0.41 | 0.74 | -0.38 | -0.07 | -0.08 | 0.00 |
| Control yeast 6 | -0.29 | -0.22 | 0.58 | 3.05 | -0.23 | -0.24 | -0.19 | -0.02 |
| Concentration (10 $\mu$M) | | | | | | | | |

As shown in Table 9, reporter gene expression was induced in the Yeast 4 and the Yeast 8 of Invention, regardless of endocrine disrupting chemical types. Thus, the response element of the present invention was found to be responsive to all the tested endocrine disrupting chemicals. The Yeast 8 of Invention is a transformant having a transcription factor of *Drosophila melanogaster* (DmMET). The response element of the present invention was found to be responsive to, in addition to a transcription factor of *Crustacea,* a transcription factor of an insect; i.e., *Drosophila melanogaster.* In contrast, reporter gene expression was induced only in systems using juvenile hormone III and 4-nonyl phenol in the control yeast 5 and the control yeast 6 each having a juvenile hormone response element of *Drosophila melanogaster* (DmJHRR), and DmJHRR did not show responsiveness to other endocrine disrupting chemicals. The results demonstrate that reporter assays involving the use of the response element of the present invention enable detection of endocrine disrupting chemicals exerting various actions.

[Test Example 5] Assay of reporter activity to antagonist substance for juvenile hormone receptors

With the use of the yeast 4 of the present invention, antagonist activity of juvenile hormone receptors to endrin, aldrin, and dieldrin was evaluated at 10 μM. The test was performed in accordance with Test Example 1, except that the test was performed in the presence of 10 nM methyl farnesoate. The results are shown in Table 10.

[Table 10]

| Tested yeast transformant | Increase in induction | | |
|---|---|---|---|
| | Endrin | Aldrin | Dieldrin |
| Yeast 4 of Invention | -0.03 | -9.02 | -12.46 |
| 0: Group treated with 10 nM methyl farnesoate Concentration (10 μM) | | | |

As shown in Table 10, the Yeast 4 of Invention was found to suppress an increase in the reporter activity caused by methyl farnesoate on all the tested substances. The results demonstrate that the use of a transformant comprising the response element of the present invention integrated thereinto enables detection of endocrine disrupting chemicals exerting antagonist activity.

[Test Example 6] Assay of reporter activity of yeast transformant to active ingredients of conventional agricultural chemicals

In accordance with Test Example 1, endocrine disrupting actions on active ingredients of conventional agricultural chemicals; i.e., mepanipyrim, benthiavalicarb isopropyl, pyribencarb, pyroxasulfone, and fenquinotrione, were assayed using the Yeast 4 of Invention. Active ingredients of conventional agricultural chemicals were tested at 100 μM. All the tested active ingredients of conventional agricultural chemicals are known to exert no endocrine disrupting actions. The results are shown in Table 11.

[Table 11]

| Tested yeast transformant | Increase in induction | | | | |
|---|---|---|---|---|---|
| | Mepanipyrim | Benthiavalicarb isopropyl | Pyribencarb | Pyroxasulfone | Fenquinotrione |
| Yeast 4 of Invention | -0.76 | -0.37 | -0.24 | -0.50 | -0.24 |
| Concentration (100 μM) | | | | | |

As shown in Table 11, reporter gene expression was not induced in the transformant comprising the response element of the present invention on all the active ingredients of conventional agricultural chemicals at 100 μM. The results demonstrate that a transformant comprising a nucleic acid encoding a receptor for endocrine disrupting chemicals having transcription factor activity, the response element according to the present invention, and a reporter gene introduced thereinto enables selective detection of endocrine disrupting chemicals.

[Test Example 7] Influence of peripheral and linker regions of core sequence on reporter activity of yeast transformant

The influence of the core sequence on reporter activity to methyl farnesoate was evaluated using the Yeasts 21, 22, 23, and 24 of Invention comprising a nucleotide sequence derived from the nucleotide sequence shown in SEQ ID NO: 2 by substitution of 3 nucleotides outside the 5' terminus of the C-box (CACGCG) in the response element; the Yeasts 25, 26, 27, and 28 of Invention comprising a nucleotide sequence derived from the nucleotide sequence shown in SEQ ID NO: 2 by substitution of 3 nucleotides outside the 3' terminus of the E-box (CACGTG) in the response element; the Yeasts 29, 30, and 31 of Invention comprising a nucleotide sequence derived from the nucleotide sequence shown in SEQ ID NO: 2 by substitution of 3 nucleotides outside the 5' terminus of the C-box (CACGCG) and 3 nucleotides outside the 3' terminus of the E-box (CACGTG) in the response element; the Yeasts 32, 33, 34, and 35 of Invention each comprising a different nucleotide sequence in a region between the C-box and the E-box (a linker region); and the Yeasts 36, 37, and 38 of Invention comprising a nucleotide sequence derived from the nucleotide sequence shown in SEQ ID NO: 2 by substitution of 3 nucleotides outside the 5' terminus of the C-box (CACGCG), 3 nucleotides outside the 3' terminus of the E-box (CACGTG), and a region between the C-box and the E-box (a linker region). Methyl farnesoate was treated at 100 nM and 10 μM. The test was performed in accordance with Test Example 1. The results are shown in Table 12.

[Table 12]

| Tested yeast transformant | Amount of juvenile hormone (methyl farnesoate) treated | |
|---|---|---|
| | 100 nM | 10 μM |
| Yeast 21 of Invention | 0.20 | 0.19 |
| Yeast 22 of Invention | 0.10 | 0.20 |
| Yeast 23 of Invention | 0.49 | 1.09 |
| Yeast 24 of Invention | 0.93 | 1.66 |
| Yeast 25 of Invention | 0.06 | 0.15 |
| Yeast 26 of Invention | 0.35 | 0.38 |
| Yeast 27 of Invention | 0.03 | 0.64 |
| Yeast 28 of Invention | 1.87 | 2.51 |
| Yeast 29 of Invention | 0.07 | 0.07 |
| Yeast 30 of Invention | 0.16 | 0.07 |
| Yeast 31 of Invention | 0.09 | 0.23 |
| Yeast 32 of Invention | 0.11 | 0.31 |
| Yeast 33 of Invention | 0.42 | 0.22 |
| Yeast 34 of Invention | 0.66 | 2.15 |
| Yeast 35 of Invention | 1.44 | 1.05 |
| Yeast 36 of Invention | 0.06 | 1.38 |
| Yeast 37 of Invention | 0.17 | 0.13 |
| Yeast 38 of Invention | 0.09 | 0.04 |

As shown in Table 12, it is apparent that reporter gene expression is more potently induced in the presence of juvenile hormone (methyl farnesoate) in the Yeasts 21, 22, 23, 24, 25, 26, 27, 28, 29, 30, 31, 32, 33, 34, 35, 36, 37, and 38 of Invention. The results demonstrate that a nucleotide sequence consisting of a total of 20 to 60 nucleotides would enable evaluation of reporter activity to methyl farnesoate, which is a juvenile hormone, with high sensitivity, even if 4 nucleotides in a region (a linker region) between the C-box (CACGCG) and the E-box (CACGCG) are kmkk (wherein k indicates G or t; and m indicates A or C) or, for example, TATT other than GCGG. Even if a nucleotide sequence in the vicinity of the nucleotide sequence shown in SEQ ID NO: 1 is different from the nucleotide sequence shown in SEQ ID NO: 2, in addition, reporter activity to juvenile hormone-like substances can also be evaluated with high sensitivity.

**Claims**

1. A nucleic acid comprising a total of 20 to 60 nucleotides comprising the nucleotide sequence shown in SEQ ID NO: 1.

2. The nucleic acid according to Claim 1, wherein arbitrary 4 nucleotides (nnnn) comprised in the nucleotide sequence shown in SEQ ID NO: 1 are kmkk, provided that k indicates g or t and m indicates a or c.

3. The nucleic acid according to Claim 1, wherein arbitrary 4 nucleotides (nnnn) comprised in the nucleotide sequence shown in SEQ ID NO: 1 are GCGG or TATT.

4. The nucleic acid according to any one of Claims 1 to 3, which comprises nucleotide sequences of given nucleotide lengths on the 3' terminal side and the 5' terminal side of the nucleotide sequence shown in SEQ ID NO: 1.

5. The nucleic acid according to Claim 1, which consists of a nucleotide sequence selected from the group consisting of SEQ ID NOs: 2, 5, 8, 34, 37, 40, 43, 46, 49, and 52.

6. A vector comprising the nucleic acid according to any one of Claims 1 to 5.

7. The vector according to Claim 6, wherein the nucleic acid is designated as a unit and a plurality of units of the nucleic acids are bound to each other.

8. The vector according to Claim 6, which comprises a reporter gene on the 3' terminal side of a sense strand of the nucleic acid.

9. A transformant comprising the nucleic acid according to any one of Claims 1 to 5 introduced into a host.

10. The transformant according to Claim 9, wherein the nucleic acid is designated as a unit and a plurality of units of the nucleic acids are bound to each other.

11. The transformant according to Claim 9, which comprises a reporter gene on the 3' terminal side of a sense strand of the nucleic acid.

12. The transformant according to Claim 9, which comprises a nucleic acid encoding a receptor for endocrine disrupting chemicals that interacts with the nucleic acid introduced thereinto.

13. The transformant according to Claim 12, wherein the receptor for endocrine disrupting chemicals is a juvenile hormone receptor of arthropods.

14. The transformant according to Claim 13, wherein the juvenile hormone receptor of arthropods is a juvenile hormone receptor of *Crustacea* or an insect.

15. The transformant according to Claim 14, wherein the juvenile hormone receptor of *Crustacea* is a juvenile hormone receptor of daphnid.

16. The transformant according to Claim 15, wherein the juvenile hormone receptor of daphnid is a protein (a) or (b):

(a) a protein consisting of the amino acid sequence shown in SEQ ID NO: 12; or
(b) a protein consisting of an amino acid sequence having 70% or higher identity to the amino acid sequence shown in SEQ ID NO: 12 and having activity of a transcription factor for a juvenile hormone receptor.

17. The transformant according to Claim 9, which further comprises a nucleic acid encoding a transcription-coupling factor introduced thereinto.

18. The transformant according to Claim 9, wherein the host is a yeast.

19. A method for evaluating a test substance comprising:

a step of bringing a test substance into contact with a transformant comprising the nucleic acid according to any one of Claims 1 to 5 and a reporter gene on the 3' terminal side of a sense strand of the nucleic acid introduced into a host and expressing a receptor for endocrine disrupting chemicals that interacts with the nucleic acid; and
a step of assaying the expression level of the reporter gene,
wherein the interaction between the test substance and the receptor for endocrine disrupting chemicals is evaluated based on the expression level of the reporter gene.

20. The method of evaluation according to Claim 19, wherein, when the expression level of the reporter gene is increased after the contact with the test substance, the test substance is determined as an agonist for the receptor for endocrine disrupting chemicals.

21. The method of evaluation according to Claim 19, wherein the test substance is brought into contact with the transformant together with at least one substance selected from the group consisting of the endocrine disrupting chemicals interacting with the receptor for endocrine disrupting chemicals, hormones, and the agonist for the receptor for endocrine disrupting chemicals, and, when the expression level of the reporter gene is lower than the expression level measured when the substance is brought into contact by itself, the test substance is determined as an agonist for the receptor for endocrine disrupting chemicals.

22. The method of evaluation according to Claim 19, wherein the nucleic acid is designated as a unit and a plurality of units of the nucleic acids are bound to each other.

23. The method of evaluation according to Claim 19, wherein a nucleic acid encoding the receptor for endocrine disrupting chemicals is introduced into the transformant.

24. The method of evaluation according to Claim 19, wherein the receptor for endocrine disrupting chemicals is a juvenile hormone receptor of arthropods.

25. The method of evaluation according to Claim 24, wherein the juvenile hormone receptor of arthropods is a juvenile hormone receptor of *Crustacea* or an insect.

26. The method of evaluation according to Claim 25, wherein the juvenile hormone receptor of *Crustacea* is a juvenile hormone receptor of daphnid.

27. The method of evaluation according to Claim 26, wherein the juvenile hormone receptor of daphnid is a protein (a) or (b):

(a) a protein consisting of the amino acid sequence shown in SEQ ID NO: 12; or
(b) a protein consisting of an amino acid sequence having 70% or higher identity to the amino acid sequence shown in SEQ ID NO: 12 and having activity of a transcription factor for a juvenile hormone receptor.

28. The method of evaluation according to Claim 19, wherein the transformant further comprises a nucleic acid encoding a transcription-coupling factor introduced thereinto.

29. The method of evaluation according to Claim 19, wherein the host is a yeast.

30. A kit for assaying endocrine disrupting chemicals comprising the vector according to Claim 8 or the transformant according to Claim 11.

INTERNATIONAL SEARCH REPORT

| | International application No. |
|---|---|
| | PCT/JP2021/016086 |

**A. CLASSIFICATION OF SUBJECT MATTER**
C12N 1/19(2006.01)i; C12N 15/113(2010.01)i; C12N 15/63(2006.01)i; C12Q 1/6897(2018.01)i
FI: C12N15/113 Z ZNA; C12N15/63 Z; C12N1/19; C12Q1/6897 Z

According to International Patent Classification (IPC) or to both national classification and IPC

**B. FIELDS SEARCHED**

Minimum documentation searched (classification system followed by classification symbols)
C12N1/19; C12N15/113; C12N15/63; C12Q1/6897

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2002-000272 A (MITSUBISHI-TOKYO PHARMACEUTICALS INC.) 08 January 2002 (2002-01-08) paragraph [0044] | 1 |
| X | 原島小夜子他，オオミジンコ Vrille 遺伝子上流の E-box/E-box 様配列を介した幼若ホルモン受容体リガンド検出酵母株の樹立，日本環境変異原学会第 49 回大会プログラム・要旨集，13 November 2020, p. 89, 06-4, entire text, (HARASHIMA, Sayoko et al., "Novel yeast reporter gene assay for detecting ligands of Daphnia magna juvenile hormone receptor via upstream E-box and E-box-like elements of the Vrille gene", Programs and Abstracts of 49th Annual Meeting of the Japanese Environmental Mutagen Society) | 1-30 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 July 2021 (05.07.2021) | 20 July 2021 (20.07.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2021/016086

C (Continuation).　DOCUMENTS CONSIDERED TO BE RELEVANT

| Category* | Citation of document, with indication, where appropriate, of the relevant passages | Relevant to claim No. |
|---|---|---|
| X | 原島小夜子他，性決定因子 Doublese×1 の発現を司る Vrille 遺伝子上流の E-box/E-box 様配列を介したオオミジンコ幼若ホルモン受容体リガンド検出酵母株の樹立, J. Toxicol. Sci., September 2020, vol. 45, supplement, S 77-S 78, 0-5, entire text, (HARASHIMA, Sayoko et al., "Establishment of reporter assay yeast strain for detecting ligands of Daphnia magna juvenile hormone receptor via upstream E-box and E-box-like elements of the Vrille gene") | 1-30 |
| A | 佐野恵梨花他，セイヨウミツバチの幼若ホルモン受容体発現レポーターアッセイ酵母の作製，日本農薬学会第 45 回大会プログラム講演要旨集, 08 March 2020, p. 128, C214, entire text, (SANO, Erika et al., "Establishment of reporter assay yeasts expressing juvenile receptor of the European honey bee Apis mellifera", Program and Lecture Abstracts of the 45th Annual Meeting of the Pesticide Science Society of Japan) | 1-30 |
| A | JP 2020-039279 A (KUMIAI CHEMICAL INDUSTRY CO., LTD.) 19 March 2020 (2020-03-19) claims, examples | 1-30 |

**INTERNATIONAL SEARCH REPORT**
Information on patent family members

International application No.

PCT/JP2021/016086

| Patent Documents referred in the Report | Publication Date | Patent Family | Publication Date |
|---|---|---|---|
| JP 2002-000272 A | 08 Jan. 2002 | (Family: none) | |
| JP 2020-039279 A | 19 Mar. 2020 | (Family: none) | |

Form PCT/ISA/210 (patent family annex) (January 2015)

## REFERENCES CITED IN THE DESCRIPTION

### Patent documents cited in the description

- JP 5754681 B

### Non-patent literature cited in the description

- **QIANYU HE et al.** Heat Shock Protein 83 (Hsp83) Facilitates Methoprene-tolerant (Met) Nuclear Import to Modulate Juvenile Hormone Signaling. *Journal of Biological Chemistry,* 2014, vol. 289, 27874-27885
- **NORIHISA TATARAZAKO et al.** Juvenile hormone agonists affect the occurrence of male Daphnia. *Chemosphere,* 2003, vol. 53, 827-833
- **KENJI TOYOTA et al.** Methyl farnesoate synthesis is necessary for the environmental sex determination in the water flea Daphnia pulex. *Journal of Insect Physiology,* 2015, vol. 80, 22-30
- **HELEN YING WANG et al.** The screening of chemicals for juvenoid-related endocrine activity using the water flea Daphnia magna. *Aquatic Toxicology,* 2005, vol. 74, 193-204
- **TOMAS A. GORR et al.** A candidate juvenoid hormone receptor cis-element in the Daphnia magna hb2 hemoglobin gene promoter. *Molecular and Cellular Endocrinology,* 2006, vol. 247, 91-102
- **NUR SYAFIQAH MOHAMAD ISHAK et al.** *Co-option of the bZIP transcription factor Vrille as the activator of Doublesex 1 in environmental sex determination of the crustacean Daphnia magna,* 2017, vol. 13, e1006953
- **HE Q et al.** *J. Biol. Chem.,* 2014, vol. 289 (40), 27874-27885
- Molecular Cloning: A Laboratory Manual
- **WOLF SS et al.** *Biotechniques,* 1996, vol. 20 (4), 568-573
- **ITO-HARASHIMA S et al.** *FEBS Open Bio.,* 2017, vol. 7 (7), 995-1008